# EUROPEAN PATENT APPLICATION

(11) **EP 4 454 592 A1**
(43) Date of publication of application: **30.10.2024**
(21) Application number: 22911105.9
(22) Date of filing: 16.12.2022
(51) Int. Cl.: A61B 34/35, B25J 3/00

(54) **SURGERY ASSISTANCE SYSTEM AND OPERATOR-SIDE DEVICE**

(30) Priority: 22.12.2021 JP 2021208663
(71) Applicant: KAWASAKI JUKOGYO KABUSHIKI KAISHA, Kobe-shi, Hyogo 650-8670 (JP)
(72) Inventor: TOJO Tsuyoshi, Hyogo 650-8670 (JP); KUNO Hirotaka, Hyogo 650-8670 (JP); MUNETO, Koji, Kobe-shi, Hyogo 650-0047 (JP)
(74) Representative: Hoffmann Eitle
(86) International application number: PCT/JP2022/046345
(87) International publication number: WO 2023/120401

(57) **Abstract**

A robotic surgical system (100) includes a surgical instrument operation unit (120) including a first board (140) to which a signal received by the surgical instrument operation unit (120) is input, and a controller (110) connected to the first board (140) by serial communication via a first wire line (141).

## Description

### Technical Field

The present disclosure relates to a robotic surgical system and an operator-side apparatus.

### Background Art

Conventionally, a robotic surgical system is disclosed. U.S. Patent Application Publication No. 2004/0243110 discloses a technology that controls movement of a surgical instrument attached to an articulated robot arm as a slave based on an operation amount received by an operation unit provided on a master control device. Grip members that are operated by the fingers of an operator are arranged on the operation unit. When the operator operates the grip members, an end effector arranged at a distal end of the surgical instrument is opened and closed. Usually, a wire line that transmits a signal from a sensor that detects an opening angle between the grip members is connected to a controller that controls the master control device.

### Prior Art

### Patent Document

Patent Document 1: U.S. Patent Application Publication No. 2004/0243110

### Summary of the Invention

A sensor other than the detection sensor that detects the opening angle between the grip members and an operation button may be arranged on the operation unit. In this case, the number of wire lines extending from the operation unit to the controller is required to correspond to the number of detection sensors, sensors other than the detection sensors, and operation buttons. The wire lines from the operation unit to the controller become thick and heavy, and the bending load increases. Thus, both a structure and a drive must be large, which affects miniaturization. Therefore, it is desired to reduce the number of wire lines extending to the controller.

The present disclosure is intended to solve the above problem. The present disclosure aims to provide a robotic surgical system and an operator-side apparatus each capable of reducing the number of wire lines extending to a controller.

A robotic surgical system according to a first aspect of the present disclosure includes a surgical instrument operation unit to receive an operation amount for a surgical instrument attached to a distal end of a robot arm, and a controller. The surgical instrument operation unit includes a first board to which a signal received by the surgical instrument operation unit is input; and the controller is connected to the first board by serial communication via a first wire line.

In the robotic surgical system according to the first aspect of the present disclosure, as described above, the controller is connected by serial communication via the first wire line to the first board to which the signal received by the surgical instrument operation unit is input. Accordingly, even when a sensor other than a detection sensor that detects an opening angle between grip members and an operation button are arranged on the surgical instrument operation unit, the first board is connected to the controller by serial communication while wiring extending from the detection sensor, the sensor other than the detection sensor, and the operation button is connected to the first board. Consequently, the number of wire lines can be reduced as compared with a case in which the detection sensor, the sensor other than the detection sensor, and the operation button are each connected to the controller. Furthermore, the length of the wire line is reduced as compared with a case in which the detection sensor, the sensor other than the detection sensor, and the operation button are each connected to the controller, and thus the influence of noise can be reduced. In addition, when the length of the wiring from the detection sensor, the sensor other than the detection sensor, and the operation button to the first board is reduced, and the length of the wire line from the first board to the controller is increased, it is particularly effective to connect the first board to the controller by serial communication in order to reduce the total length of the wire lines used.

An operator-side apparatus according to a second aspect of the present disclosure includes a surgical instrument operation unit to receive an operation amount for a surgical instrument attached to a distal end of a robot arm, and a controller. The surgical instrument operation unit includes a board to which a signal received by the surgical instrument operation unit is input, and the controller is connected to the board by serial communication via a wire line.

In the operator-side apparatus according to the second aspect of the present disclosure, as described above, the controller is connected by serial communication via the wire line to the first board to which the signal received by the surgical instrument operation unit is input. Accordingly, even when a sensor other than a detection sensor that detects an opening angle between grip members and an operation button are arranged on the surgical instrument operation unit, the first board is connected to the controller by serial communication while wiring extending from the detection sensor, the sensor other than the detection sensor, and the operation button is connected to the first board. Consequently, it is possible to provide the operator-side apparatus capable of reducing the number of wire lines extending from the detection sensor, the sensor other than the detection sensor, and the operation button to the controller. Furthermore, the length of the wire line extending from the sensor other than the detection sensor and the operation button is reduced as compared with a case in which the detection sensor, the sensor other than the detection sensor, and the operation button are each connected to the controller, and thus the influence of noise can be reduced. In addition, it is particularly effective to connect the first board to the controller by serial communication in order to reduce the total length of the wire lines used.

According to the present disclosure, the number of wire lines extending to the controller can be reduced.

### Brief Description of the Drawings

FIG. 1 is a diagram showing the configuration of a robotic surgical system according to an embodiment.
FIG. 2 is a diagram showing the configuration of a robot arm according to the embodiment.
FIG. 3 is a diagram showing a pair of forceps.
FIG. 4 is a perspective view showing the configuration of an arm operation unit according to the embodiment.
FIG. 5 is a diagram for illustrating translational movement of the robot arm.
FIG. 6 is a diagram for illustrating rotational movement of the robot arm.
FIG. 7 is a diagram showing an operation handle according to the embodiment.
FIG. 8 is a diagram showing the configuration of the operation handle according to the embodiment.
FIG. 9 is a diagram showing the configuration of foot pedals according to the embodiment.
FIG. 10 is a control block diagram of the robotic surgical system according to the embodiment.
FIG. 11 is a control block diagram of a remote control apparatus according to the embodiment.
FIG. 12 is a control block diagram of an operation unit according to the embodiment.
FIG. 13 is a sectional view taken along the line 200-200 in FIG. 7.

### Modes for Carrying Out the Invention

### Configuration of Robotic Surgical System

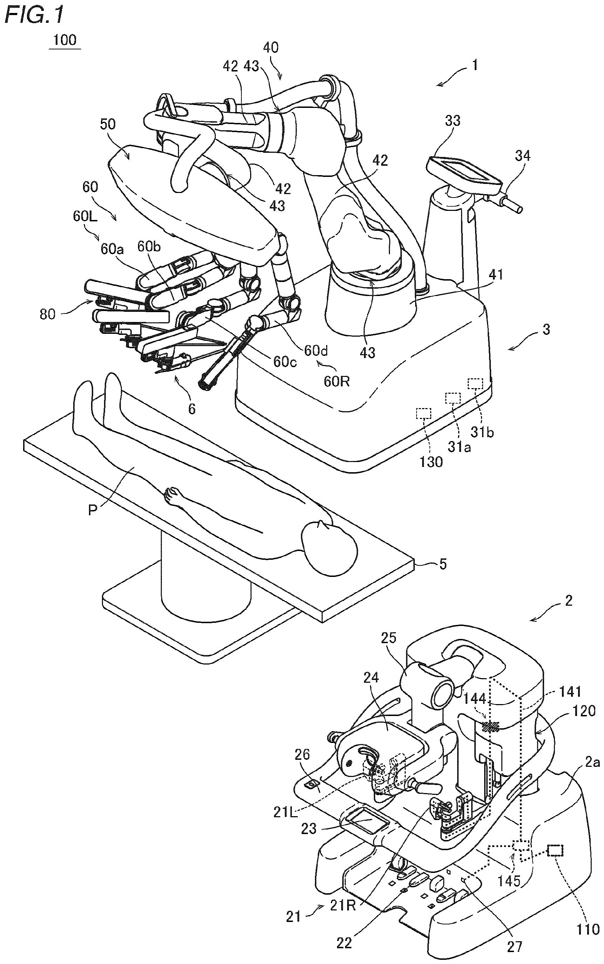

The configuration of a robotic surgical system 100 according to the present embodiment is now described with reference to FIGS. 1 to 13. The robotic surgical system 100 includes a surgical robot 1 and a remote control apparatus 2.

The surgical robot 1 is a patient P-side apparatus. The surgical robot 1 includes a medical cart 3, and is movable. The surgical robot 1 is arranged in an operating room. The remote control apparatus 2 is an operator-side apparatus for operating the surgical robot 1. The remote control apparatus 2 is spaced apart from the surgical robot 1, and the surgical robot 1 is remotely controlled by the remote control apparatus 2. An operator such as a doctor inputs a command to the remote control apparatus 2 to cause the surgical robot 1 to perform a desired operation. The remote control apparatus 2 transmits the input command to the surgical robot 1. The surgical robot 1 operates based on the received command. The surgical robot 1 is arranged in the operating room that is a sterilized sterile field.

### Configuration of Surgical Robot

As shown in FIG. 1, the surgical robot 1 includes the medical cart 3, a positioner 40, an arm base 50, a plurality of robot arms 60, and arm operation units 80.

The medical cart 3 moves the positioner 40. The medical cart 3 includes an input 33. The input 33 receives operations to move the positioner 40, the arm base 50, and the plurality of robot arms 60 or change their postures mainly in order to prepare for surgery before the surgery. The medical cart 3 includes an operation handle 34 that receives an operator's steering operation.

The positioner 40 includes a 7-axis articulated robot, for example. The positioner 40 is arranged on the medical cart 3. The positioner 40 adjusts the position of the arm base 50. The positioner 40 moves the position of the arm base 50 three-dimensionally.

The positioner 40 includes a base 41 and a plurality of links 42 coupled to the base 41. The plurality of links 42 are coupled to each other by joints 43.

The arm base 50 is attached to a distal end of the positioner 40. A proximal end of each of the plurality of robot arms 60 is attached to the arm base 50. Each of the plurality of robot arms 60 is able to take a folded and stored posture. The arm base 50 and the plurality of robot arms 60 are covered with sterile drapes and used. Moreover, each of the robot arms 60 supports a surgical instrument 4.

The plurality of robot arms 60 are arranged. Specifically, four robot arms 60a, 60b, 60c, and 60d are arranged. The robot arms 60a, 60b, 60c, and 60d have the same or similar configurations as each other.

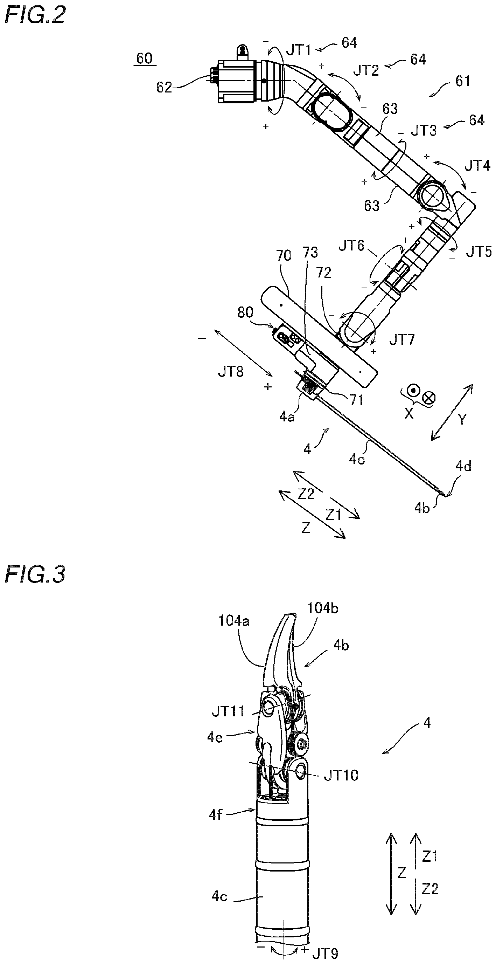

As shown in FIG. 2, each robot arm 60 includes an arm portion 61, a first link 72, a second link 73, and a translation mechanism 70. The robot arm 60 includes JT1 to JT7 axes as rotation axes and a JT8 axis as a linear motion axis. The JT1 to JT7 axes are rotation axes of joints 64 of the arm portion 61. Furthermore, the JT7 axis is a rotation axis of the first link 72. The JT8 axis is a linear motion axis along which the translation mechanism 70 moves the second link 73 relative to the first link 72 along a Z direction.

The arm portion 61 includes a 7-axis articulated robot arm. The first link 72 is arranged at a distal end of the arm portion 61. An arm operation unit 80 is attached to the second link 73. The translation mechanism 70 is arranged between the first link 72 and the second link 73. A holder 71 that holds the surgical instrument 4 is arranged on the second link 73.

The surgical instrument 4 is attached to a distal end of each of the plurality of robot arms 60. The surgical instrument 4 includes a replaceable instrument, an endoscope 6 to capture an image of a surgical site, etc. The surgical instrument 4 as the instrument includes a driven unit 4a, a pair of forceps 4b, and a shaft 4c that connects the driven unit 4a to the pair of forceps 4b. The driven unit 4a, the shaft 4c, and the pair of forceps 4b are arranged along the Z direction.

As shown in FIG. 1, the endoscope 6 is attached to the distal end of one of the plurality of robot arms 60, such as the robot arm 60c, and surgical instruments 4 other than the endoscope 6 are attached to the distal ends of the remaining robot arms 60a, 60b, and 60d, for example. The endoscope 6 is attached to one of two robot arms 60b and 60c arranged in the center among the four robot arms 60 arranged adjacent to each other.

### Configuration of Instrument

As shown in FIG. 3, the pair of forceps 4b is provided at a distal end of the instrument, for example. At the distal end of the instrument, in addition to the pair of forceps 4b, a pair of scissors, a grasper, a needle holder, a microdissector, a stable applier, a tacker, a suction cleaning tool, a snare wire, a clip applier, etc. are arranged as instruments having joints. At the distal end of the instrument, a cutting blade, a cautery probe, a washer, a catheter, a suction orifice, etc. are arranged as instruments having no joint.

The pair of forceps 4b includes a first support 4e that supports the proximal end sides of jaw members 104a and 104b on the distal end side such that the proximal end sides of the jaw members 104a and 104b are rotatable about a JT11 axis, and a second support 4f that supports the proximal end side of the first support 4e on the distal end side such that the proximal end side of the first support 4e is rotatable about a JT10 axis. The shaft 4c rotates about a JT9 axis. The jaw members 104a and 104b pivot about the JT11 axis to open and close.

As shown in FIG. 2, the arm operation unit 80 is attached to the robot arm 60. Specifically, the arm operation unit 80 is attached to the second link 73.

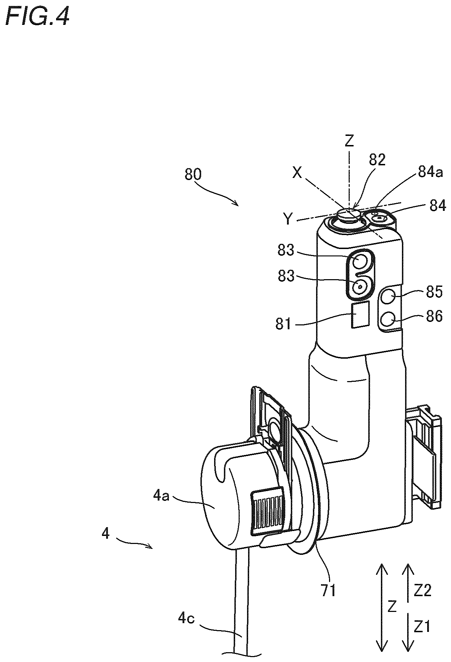

As shown in FIG. 4, the arm operation unit 80 includes an enable switch 81, a joystick 82, and linear switches 83, a pivot button 85, an adjustment button 86, a mode switching button 84, and a mode indicator 84a.

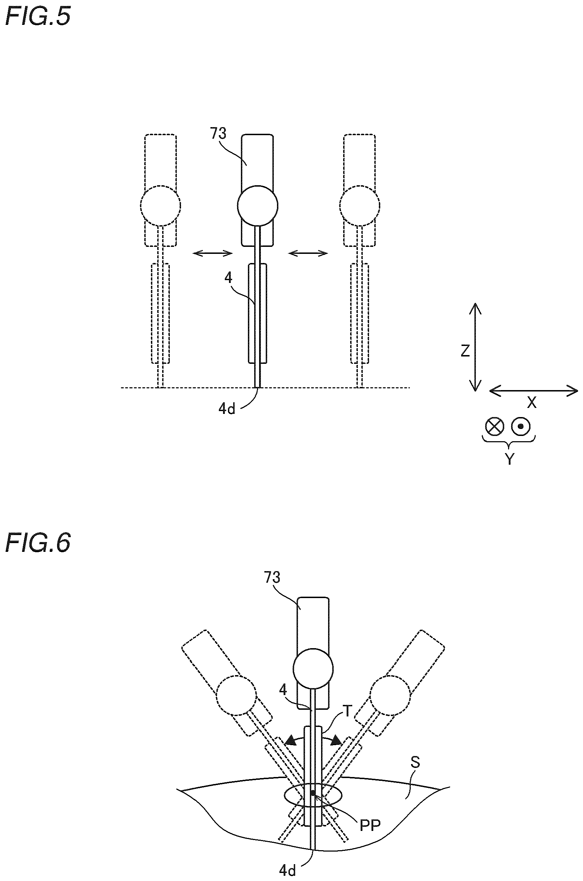

The enable switch 81 is a switch to enable or disable movement of the robot arm 60 in response to the joystick 82 and the linear switches 83. The joystick 82 is an operation tool to control movement of the surgical instrument 4 by the robot arm 60. The linear switches 83 are switches to move the surgical instrument 4 in a direction along the longitudinal direction of the surgical instrument 4. The pivot button 85 is a button to set a pivot position PP that serves as a fulcrum for movement of the surgical instrument 4 attached to the robot arm 60. The adjustment button 86 is a button to optimize the position of the robot arm 60. The mode switching button 84 is a button to switch between a mode for translationally moving the surgical instrument 4 as shown in FIG. 5 and a mode for rotationally moving the surgical instrument 4 as shown in FIG. 6. The mode indicator 84a indicates a selected mode.

### Remote Control Apparatus

As shown in FIG. 1, the remote control apparatus 2 is arranged inside or outside the operating room, for example. The remote control apparatus 2 includes a main body 2a, an operation unit 120, foot pedals 22, a touch panel 23, a monitor 24, a support arm 25, a support bar 26, and foot detectors 27. The operation unit 120 includes an operation handle for the operator such as a doctor to input a command. The operation unit 120 is an example of a surgical instrument operation unit.

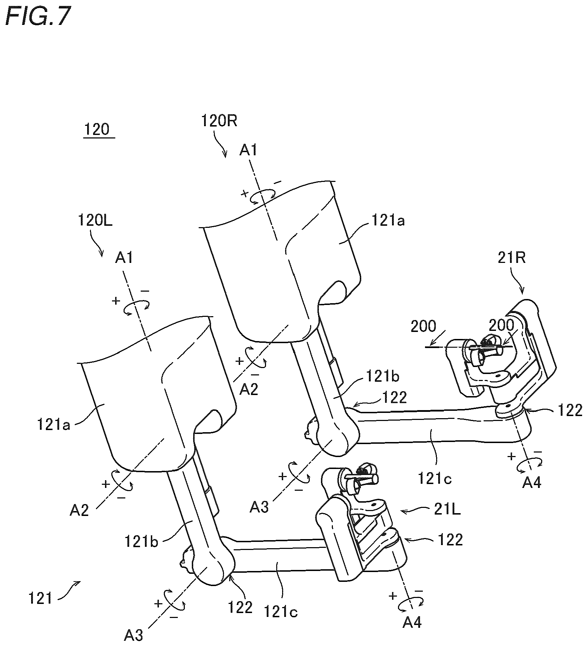

As shown in FIG. 1, the operation unit 120 is supported by the main body 2a. As shown in FIG. 7, the operation unit 120 receives an operation amount for the surgical instrument 4. The operation unit 120 includes an operation unit 120L located on the left side as viewed from the operator such as a doctor and operated by the left hand of the operator, and an operation unit 120R located on the right side and operated by the right hand of the operator. The configurations of the operation unit 120L and the operation unit 120R are the same as or similar to each other.

The operation unit 120 includes a substantially L-shaped arm 121 and an operation handle 21. The arm 121 includes a first link 121a, a second link 121b, and a third link 121c. The upper end side of the first link 121a is attached to the main body 2a such that the first link 121a is rotatable about an A1 axis along a vertical direction. The upper end side of the second link 121b is attached to the lower end side of the first link 121a such that the second link 121b is rotatable about an A2 axis along a horizontal direction. A first end side of the third link 121c is attached to the lower end side of the second link 121b such that the third link 121c is rotatable about an A3 axis along the horizontal direction. The operation handle 21 is attached to a second end side of the third link 121c such that the operation handle 21 is rotatable about an A4 axis. The links are connected by joints 122.

The arm 121 supports the operation handle 21. The arm 121 supports the operation handle 21 such that the operation handle 21 is movable within a predetermined three-dimensional operation range. Specifically, the arm 121 supports the operation handle 21 such that the operation handle 21 is movable in an upward-downward direction, a right-left direction, and a forward-rearward direction. The robot arm 60 is moved three-dimensionally so as to correspond to a three-dimensional operation on the arm 121.

As shown in FIG. 7, the operation handle 21 includes an operation handle 21L located on the left side as viewed from the operator such as a doctor and operated by the left hand of the operator, and an operation handle 21R located on the right side and operated by the right hand of the operator.

The operation handle 21 includes a link 21a, a link 21b, a link 21c, and a link 21d operated by the operator such as a doctor. The link 21a rotates about the A4 axis. The link 21b rotates about an A5 axis with respect to the link 21a. The link 21c rotates about an A6 axis with respect to the link 21b. The link 21d rotates about an A7 axis with respect to the link 21c. The links are connected by joints 122. The link 21a, the link 21b, the link 21c, and the link 21d are examples of a fourth link, a third link, a second link, and a first link, respectively.

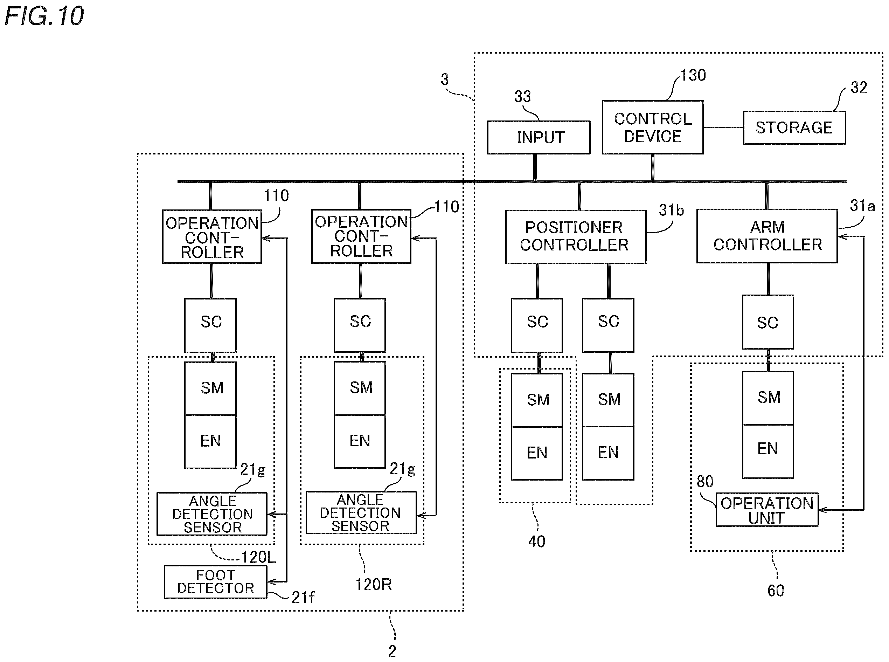

The operation handle 21 includes a pair of grip members 21f that are opened and closed by the operator. The grip members 21f each include an elongated plate-shaped lever member, and proximal ends of the pair of grip members 21f are rotatably connected to a proximal end G1 of the link 21d. Cylindrical finger insertion portions 21e are provided on the grip members 21f. The operator inserts their fingers into a pair of finger insertion portions 21e to operate the operation handle 21. The proximal ends of the pair of grip members 21f are connected to the link 21d, and an angle between the pair of grip members 21f is increased or decreased such that an opening angle between the jaw member 104a and the jaw member 104b is changed. A magnet is provided on one of the grip members 21f, and a Hall sensor is provided on the link 21d. When the operator opens and closes the grip members 21f, the magnet and the Hall sensor function as an angle detection sensor 21g, as shown in FIG. 10, and the Hall sensor outputs the opening angle. As the angle detection sensor 21g, the Hall sensor may be provided on the grip member 21f, and the magnet may be provided on the link 21d. Alternatively, the magnet or the Hall sensor may be provided as the angle detection sensor 21g on both the grip members 21f.

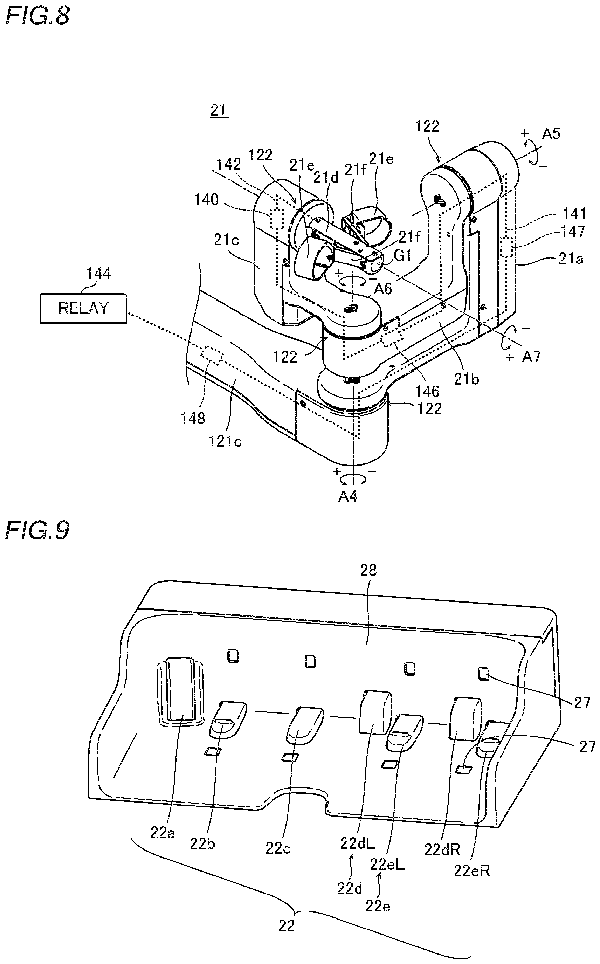

As shown in FIG. 9, a plurality of foot pedals 22 are provided to execute functions of the surgical instrument 4. The plurality of foot pedals 22 are arranged on a base 28. The foot pedals 22 include a switching pedal 22a, a clutch pedal 22b, a camera pedal 22c, an incision pedal 22d, and a coagulation pedal 22e. The switching pedal 22a, the clutch pedal 22b, the camera pedal 22c, the incision pedal 22d, and the coagulation pedal 22e are operated by the foot of the operator. The incision pedal 22d includes an incision pedal 22dR for a right robot arm 60, and an incision pedal 22dL for a left robot arm 60. The coagulation pedal 22e includes a coagulation pedal 22eR for the right robot arm 60 and a coagulation pedal 22eL for the left robot arm 60.

The switching pedal 22a switches a robot arm 60 to be operated by the operation handle 21. The clutch pedal 22b performs a clutch operation to temporarily disconnect an operation connection between the robot arm 60 and the operation handle 21. While the clutch pedal 22b is being pressed by the operator, an operation by the operation handle 21 is not transmitted to the robot arm 60. While the camera pedal 22c is being pressed by the operator, the operation handle 21 can operate a robot arm 60 to which the endoscope 6 is attached. While the incision pedal 22d or the coagulation pedal 22e is being pressed by the operator, an electrosurgical device is activated.

The foot detectors 27 detect the foot of the operator that operates the foot pedals 22. The foot detectors 27 detect the foot that hovers above their corresponding foot pedals 120. The foot detectors 27 are arranged on the base 28.

As shown in FIG. 1, the monitor 24 is a scope-type display that displays an image captured by the endoscope 6. The support arm 25 supports the monitor 24 so as to align the height of the monitor 24 with the height of the face of the operator such as a doctor. The touch panel 23 is arranged on the support bar 26. When the head of the operator is detected by a sensor provided in the vicinity of the monitor 24, the surgical robot 1 can be operated by the remote control apparatus 2. The operator operates the operation unit 120 and the foot pedals 22 while visually recognizing an affected area on the monitor 24. Thus, a command is input to the remote control apparatus 2. The command input to the remote control apparatus 2 is transmitted to the surgical robot 1.

### Configuration of Control System

As shown in FIG. 10, the robotic surgical system 100 includes a control device 130, an arm controller 31a, a positioner controller 31b, and operation controllers 110. The operation controllers 110 are examples of a controller.

The control device 130 is accommodated in the medical cart 3 to communicate with the arm controller 31a, the positioner controller 31b, and the operation controllers 110. The control device 130 controls the arm controller 31a, the positioner controller 31b, and the operation controllers 110. The control device 130 is connected to the arm controller 31a, the positioner controller 31b, the operation controllers 110, and the input 33 through a LAN, for example. The control device 130 is placed inside the medical cart 3.

The arm controller 31a is arranged for each of the plurality of robot arms 60.

The robot arm 60 includes servomotors SM, encoders EN, and speed reducers so as to correspond to a plurality of joints 64 of the robot arm 60. The arm controller 31a is arranged inside the medical cart 3 as shown in FIG. 1, and servo controllers SC are arranged adjacent to the arm controller 31a inside the medical cart 3. That is, the same number of arm controllers 31a and servo controllers SC as the plurality of robot arms 60 are placed inside the medical cart 3.

The positioner controller 31b and servo controllers SC are placed in the medical cart 3. The positioner controller 31b controls the positioner 40 and the medical cart 3. The servo controllers SC control servomotors SM of the positioner 40 and servomotors SM of the medical cart 3. The servomotors SM, encoders EN, and speed reducers are provided in the positioner 40 so as to correspond to a plurality of joints 43 of the positioner 40. The servomotors SM that drive a plurality of front wheels of the medical cart 3, encoders EN, speed reducers, and brakes are placed in the medical cart 3.

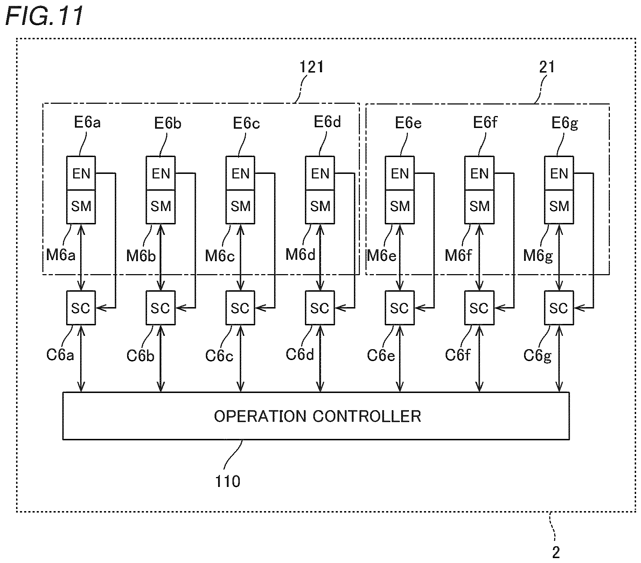

As shown in FIG. 11, the operation unit 120 includes servomotors M6a, M6b, M6c, M6d, M6e, M6f, and M6g corresponding to the A1, A2, A3, A4, A5, A6, and A7 rotation axes, respectively. Servo controllers C6a, C6b, C6c, C6d, C6e, C6f, and C6g are provided adjacent to each operation controller 110 inside the main body 2a of the remote control apparatus 2 to control the servomotors. Encoders E6a, E6b, E6c, E6d, E6e, E6f, and E6g that detect rotation angles of the servomotors are electrically connected to the servo controllers, respectively. The servomotors and the encoders are provided in each of the operation unit 120L and the operation unit 120R. Furthermore, the servo controllers and the operation controller are provided so as to correspond to each of the operation unit 120L and the operation unit 120R.

The control device 130 controls the servomotors to generate torques that cancel gravitational torques generated on the rotation axes of the servomotors according to the posture of the operation unit 120, via the operation controllers 110. Thus, the operator can operate the operation unit 120 with a relatively small force.

The control device 130 controls the servomotors to generate torques on the rotation axes of the servomotors according to an operation on the operation unit 120, via the operation controllers 110 and assist the operation of the operator. Thus, the operator can operate the operation unit 120 with a relatively small force.

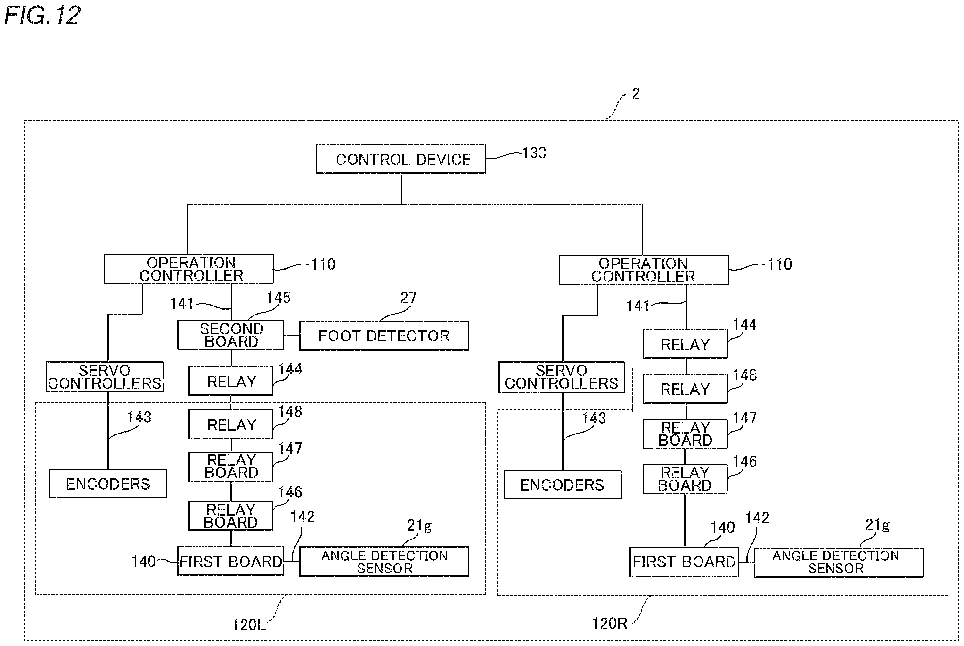

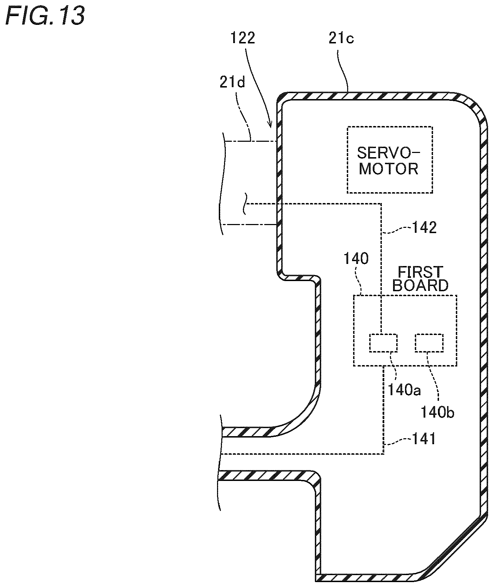

In the present embodiment, as shown in FIG. 12, first boards 140 to which signals received by the operation unit 120 are input are provided in the operation unit 120. Each operation controller 110 is connected to a corresponding first board 140 by serial communication via a first wire line 141. As shown in FIG. 13, a connector 140a, an IC 140b, etc. are placed on the first board 140. The first wire line 141 includes one transmission path. The operation controller 110 is connected to the first board 140 by means of a communication network that allows information to be shared between the operation controller 110 and the first board 140 by using serial communication. The first boards 140 are examples of a board. The first wire line 140 is an example of a wire line.

In the present embodiment, as shown in FIG. 12, an opening angle between the pair of grip members 21f is input as an analog signal to a corresponding first board 140. Specifically, an analog signal from the angle detection sensor 21g is input to the connector 140a of the first board 140.

In the present embodiment, as shown in FIG. 13, the first board 140 is placed inside the link 21c. Flexible printed wiring 142 extending from the link 21d is connected to the first board 140. Thus, the only joint 122 through which the flexible printed wiring 142 to which the signal from the angle detection sensor 21g is transmitted passes is the joint 122 that connects the link 21d to the link 21c.

In the present embodiment, as shown in FIG. 8, the operation controller 110 is connected to the first board 140 by serial communication via the first wire line 141 through the insides of the link 21c in which the first board 140 is placed, the link 21b, the link 21a, and the arm 121 shown in FIG. 7.

In the present embodiment, as shown in FIG. 1, each operation controller 110 is arranged inside the main body 2a. The operation controller 110 is connected to the first board 140 by serial communication via the first wire line 141 through the insides of the operation unit 120 and the main body 2a. Specifically, the first board 140 is inserted into the main body 2a via the proximal end side of the operation unit 120, and is connected to the operation controller 110 inside the main body 2a.

In the present embodiment, as shown in FIG. 12, the operation controller 110 is connected to the first board 140 by serial communication via the first wire line 141 separately from communication paths between the operation controller 110 and the encoders E6a, E6b, E6c, E6d, E6e, E6f, and E6g that detect the movement amounts of the servomotors M6a, M6b, M6c, M6d, M6e, M6f, and M6gf. Specifically, each encoder is connected to the operation controller 110 by serial communication via a second wire line 143. Furthermore, a bus connection is established between the encoders. Moreover, the encoders are connected to the operation controller 110 via the servo controllers. The servomotors M6a, M6b, M6c, M6d, M6e, M6f and M6gf are examples of a drive. The encoders E6a, E6b, E6c, E6d, E6e, E6f, and E6g are examples of a detector.

In the present embodiment, the operation controller 110 is connected to the first board 140 by serial communication via a second board 145, a relay 144, a relay 148, a relay board 147, and a relay board 146. As shown in FIG. 1, the relay 144 is arranged on the proximal end side of the operation unit 120. Although the relay 144 and the relay 148 are connectors, they may be relay boards including connectors, for example.

In the present embodiment, as shown in FIG. 8, the first board 140 is connected to the relay 144 by serial communication via the relay board 146, the relay board 147, and the relay 148. Specifically, the relay board 146 is arranged inside the link 21b. The relay board 147 is arranged inside the link 21a. The relay 148 is arranged inside the third link 121c. The first board 140 is connected to the relay board 146 via the first wire line 141 including flexible printed wiring. The relay board 146 is connected to the relay board 147 via the first wire line 141 including flexible printed wiring. The relay board 147 is connected to the relay 148 via the first wire line 141 including flexible printed wiring. The relay 148 is connected to the relay 144 via the first wire line 141 including cable wiring. The relay 144 is connected to the second board 145 shown in FIG. 1 via the first wire line 141 including cable wiring. The relay board 146 and the relay board 147 include connectors. The relay 148 does not include a board. The relay 148 may be connected to the relay 144 via the first wire line 141 including flexible printed wiring. The relay board 146 and the relay board 147 are examples of a "first relay board" and a "second relay board", respectively. The flexible printed wiring refers to a flexible printed circuit (FPC). The cable wiring is a wiring structure other than flexible printed wiring, and refers, for example, to a cable covered with an outer jacket made of polyvinyl chloride resin, polyethylene resin, ethylene tetrafluoroethylene (ETFE) resin, or the like. The cable wiring is sometimes referred to as a robot cable. A flexible flat cable (FFC) may be used as the flexible printed wiring.

In the present embodiment, as shown in FIG. 12, the second board 145 to which signals from the foot detectors 27 are input is arranged. In the operation unit 120L, the operation controller 110 is connected to the second board 145 by serial communication via the first wire line 141 including cable wiring. In the operation unit 120R, the operation controller 110 is connected to the relay 144 by serial communication via the first wire line 141 including cable wiring. As shown in FIG. 1, the foot detectors 27 are arranged on the base 28, and the second board 145 is arranged inside the main body 2a. The signals from the foot detectors 27 are input only to the operation unit 120L. The signals from the foot detectors 27 may be input only to the operation unit 120R. The operation controller 110 may be connected to the relay 144 by serial communication via flexible printed wiring.

### Advantages of Present Embodiment

The operation controller 110 is connected by serial communication via the first wire line 141 to the first board 140 to which a signal received by the operation unit 120 is input. Accordingly, even when a sensor other than the angle detection sensor 21g that detects the opening angle between the grip members 21f and an operation button are arranged on the operation unit 120, the first board 140 is connected to the operation controller 110 by serial communication while the flexible printed wiring 142 extending from the angle detection sensor 21g, the sensor other than the angle detection sensor 21g, and the operation button is connected to the first board 140. Consequently, the number of wire lines can be reduced as compared with a case in which the angle detection sensor 21g, the sensor other than the angle detection sensor 21g, and the operation button are each connected to the operation controller 110. Furthermore, the influence of noise can be reduced by converting an analog signal into serial communication in the first board 140 that is placed in the vicinity of the angle detection sensor 21g, the sensor other than the angle detection sensor 21g, and the operation button. In addition, when the length of the wiring from the angle detection sensor 21g, the sensor other than the angle detection sensor 21g, and the operation button to the first board 140 is reduced, and the length of the wire line from the first board 140 to the operation controller 110 is increased, it is particularly effective to connect the operation controller 110 to the first board 140 by serial communication via the first wire line 141 in order to reduce the total length of the wire lines used.

The operation controller 110 is connected to the first board 140 by serial communication via the first wire line 141 separately from the communication paths between the encoders E6a, E6b, E6c, E6d, E6e, E6f, and E6g that detect the movement amounts of the servomotors M6a, M6b, M6c, M6d, M6e, M6f, and M6g and the operation controller 110. Accordingly, interference between a signal from the operation unit 120 and a signal from each encoder can be reduced or prevented.

The encoders E6a, E6b, E6c, E6d, E6e, E6f, and E6g are connected to the operation controller 110 by serial communication via the second wire line 143. Accordingly, even when a plurality of encoders are arranged, the number of wire lines extending to the operation controller 110 can be reduced while interference between the signal from the operation unit 120 and the signal from each encoder is reduced or prevented.

The operation controller 110 is connected to the first board 140 by serial communication through the inside of the operation unit 120. Accordingly, the first board 140 and the operation controller 110 are connected to each other via the first wire line 141, and the number of wire lines is reduced. Thus, an increase in the size of the operation handle 21 can be reduced or prevented.

The operation controller 110 is connected to the first board 140 by serial communication through the relay 148. Accordingly, the relay 148 is arranged between portions to be separated from each other such that the portions to be separated from each other can be separated integrally with the firs twire line 141. Consequently, the burden of work such as reconnecting the first wire line 141 can be reduced. Furthermore, the type of wire line by which a serial communication connection is established through the relay 148 can be changed. For example, cable wiring is connected to flexible printed wiring through the relay such that the flexible printed wiring can be arranged in a movable portion. When there are a plurality of movable portions, a plurality of relays may be arranged, and flexible printed wiring may be connected to another flexible printed wiring through one of the relays.

The first board 140 is serially connected to at least one of the operation controller 110 or the relay 148 via the first wire line 141 including the flexible printed wiring. Accordingly, obstruction of rotation of each link of the operation handle 21 by the first wire line 141 can be reduced or prevented.

An analog signal from the angle detection sensor 21g that detects the opening angle between the pair of grip members 21f is input to the first board 140. Noise has a relatively large influence on an analog signal. Furthermore, as the length of the wire line increases, the influence of noise increases. Therefore, when an analog signal is transmitted from the angle detection sensor 21g to the first board 140, the first board 140 is arranged inside the link 21c adjacent to the link 21d in which the Hall sensor of the angle detection sensor 21g is arranged, and thus the length of the flexible printed wiring 142 to which the analog signal is transmitted and that is located between the angle detection sensor 21g and the first board 140 is reduced. Thus, the influence of noise on the analog signal can be reduced.

The angle detection sensor 21g is serially connected to the first board 140 via the first wire line 141 including the flexible printed wiring. Accordingly, obstruction of rotation of each link of the operation handle 21 by the first wire line 141 can be reduced or prevented.

The operation controller 110 is connected to the first board 140 by serial communication via the first wire line 141 through the insides of the link 21c in which the first board 140 is placed, the link 21b, the link 21a, and the arm 121. Accordingly, the number of wire lines between the first board 140 and the operation controller 110 is reduced, and thus an increase in the thicknesses of the link 21c, the link 21b, the link 21a, and the arm 121 can be reduced or prevented.

The first board 140 is connected to the relay board 146 via the first wire line 141 including the flexible printed wiring, the relay board 146 is connected to the relay board 147 via the first wire line 141 including the flexible printed wiring, and the relay board 147 is connected to the relay 148 via the first wire line 141 including the flexible printed wiring. Accordingly, the first wire line 141 including the flexible printed wiring is arranged between the first board 140 and the relay 148, and thus obstruction of rotation of each link of the operation handle 21 by the first wire line 141 can be reduced or prevented.

The operation controller 110 is connected to the first board 140 by serial communication via the first wire line 141 through the insides of the operation unit 120 and the main body 2a. Accordingly, a distance between the operation unit 120 and the operation controller 110 becomes relatively large, and thus it is particularly effective to connect the operation controller 110 to the first board 140 by serial communication via the first wire line 141 in order to reduce the influence of noise.

The operation controller 110 is connected to the second board 145 by serial communication via the first wire line 141. Accordingly, unlike a case in which the operation controller 110 is connected to the first board 140 and the second board 145 via separate wire lines, the number of wire lines can be reduced.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the example in which the operation controller 110 is placed in the remote control apparatus 2 has been shown in the aforementioned embodiment, the present disclosure is not limited to this. For example, the operation controller 110 may be placed in a portion other than the remote control apparatus 2.

While the example in which the first board 140 is placed in the link 21c has been shown in the aforementioned embodiment, the present disclosure is not limited to this. For example, the first board 140 may be placed in the link 21d.

While the example in which the encoders E6a, E6b, E6c, E6d, E6e, E6f, and E6g are connected to the operation controller 110 by serial communication via the second wire line 143 has been shown in the aforementioned embodiment, the present disclosure is not limited to this. For example, a communication method between each encoder and the operation controller 110 may be a communication method other than serial communication.

While the example in which the control device 130 is placed in the medical cart 3 has been shown in the aforementioned embodiment, the present disclosure is not limited to this. For example, the control device 130 may be placed outside the medical cart 3.

While the example in which the operation controller 110 is connected to the first board 140 by serial communication through the insides of the operation handle 21 and the arm 121 has been shown in the aforementioned embodiment, the present disclosure is not limited to this. For example, the operation controller 110 is connected to the first board 140 by serial communication through the outsides of the operation handle 21 and the arm 121.

While the example in which the operation controller 110 is connected to the first board 140 by serial communication through the relay 144 has been shown in the aforementioned embodiment, the present disclosure is not limited to this. For example, the operation controller 110 may be directly connected to the first board 140 without using the relay 144.

While the example in which the opening angle between the pair of grip members 21f is input as an analog signal to the first board 140 has been shown in the aforementioned embodiment, the present disclosure is not limited to this. For example, an acceleration sensor may be placed on the operation handle 21, and a signal from the acceleration sensor may be input to the first board 140 in addition to the analog signal of the opening angle between the pair of grip members 21f.

While the example in which four robot arms 60 are provided has been shown in the aforementioned embodiment, the present disclosure is not limited to this. In the present disclosure, the number of robot arms 60 may be any number as long as at least one robot arm 60 is provided.

While the example in which each of the arm portion 61 and the positioner 40 includes a 7-axis articulated robot has been shown in the aforementioned embodiment, the present disclosure is not limited to this. For example, each of the arm portion 61 and the positioner 40 may include an articulated robot having an axis configuration other than the 7-axis articulated robot. The axis configuration other than the 7-axis articulated robot includes six axes or eight axes, for example.

While the example in which the surgical robot 1 includes the medical cart 3, the positioner 40, and the arm base 50 has been shown in the aforementioned embodiment, the present disclosure is not limited to this. For example, the surgical robot 1 may not include the medical cart 3, the positioner 40, or the arm base 50, but may include only the robot arms 60.

### Aspects

It will be appreciated by those skilled in the art that the exemplary embodiments described above are specific examples of the following aspects.

### (Item 1)

A robotic surgical system comprising:
a surgical instrument operation unit to receive an operation amount for a surgical instrument attached to a distal end of a robot arm; and
a controller; wherein
the surgical instrument operation unit includes a first board to which a signal received by the surgical instrument operation unit is input; and
   the controller is connected to the first board by serial communication via a first wire line.

### (Item 2)

The robotic surgical system according to item 1, wherein
the surgical instrument operation unit includes a joint and a drive provided in the joint; and
the controller is connected to the first board by serial communication via the first wire line separately from a signal communication path between a detector operable to detect a movement amount of the drive and the controller.

### (Item 3)

The robotic surgical system according to item 2, wherein the detector is connected to the controller by serial communication via a second wire line.

### (Item 4)

The robotic surgical system according to any one of items 1 to 3, wherein the controller is connected to the first board by serial communication through an inside of the surgical instrument operation unit.

### (Item 5)

The robotic surgical system according to any one of items 1 to 4, wherein the controller is connected to the first board by serial communication through a relay.

### (Item 6)

The robotic surgical system according to item 5, wherein the relay is serially connected to at least one of the controller or the first board via the first wire line including flexible printed wiring.

### (Item 7)

The robotic surgical system according to any one of items 1 to 6, wherein
the surgical instrument operation unit includes a pair of grip members that are opened and closed by an operator, and an angle detection sensor to detect an opening angle between the pair of grip members; and
the first board is operable to receive an analog signal from the angle detection sensor.

### (Item 8)

The robotic surgical system according to item 7, wherein the angle detection sensor is serially connected to the first board via the first wire line including flexible printed wiring.

### (Item 9)

The robotic surgical system according to item 7 or 8, wherein
the surgical instrument operation unit further includes:
   a first link on which the pair of grip members are placed; and
   a second link connected to the first link; and
the first board is placed inside the first link or the second link.

### (Item 10)

The robotic surgical system according to item 9, wherein
the surgical instrument operation unit further includes:
   a third link connected to the second link;
   a fourth link connected to the third link; and
   an arm to support the fourth link; and
the controller is connected to the first board by serial communication via the first wire line through insides of the first link or the second link in which the first board is placed, the third link, the fourth link, and the arm.

### (Item 11)

The robotic surgical system according to item 10, further comprising:
a first relay board in the third link;
a second relay board in the fourth link; and
a relay placed in the arm; wherein
the first board is connected to the first relay board via the first wire line including flexible printed wiring;
the first relay board is connected to the second relay board via the first wire line including the flexible printed wiring; and
the second relay board is connected to the relay via the first wire line including the flexible printed wiring.

### (Item 12)

The robotic surgical system according to any one of items 1 to 11, further comprising:
a main body to support the surgical instrument operation unit; wherein
the controller is placed inside the main body; and
the controller is connected to the first board by serial communication via the first wire line through insides of the surgical instrument operation unit and the main body.

### (Item 13)

The robotic surgical system according to any one of items 1 to 12, further comprising:
a foot pedal operated by a foot of an operator;
a foot detector to detect the foot of the operator that operates the foot pedal; and
a second board to which a signal from the foot detector is input; wherein
the controller is connected to the second board by serial communication via the first wire line.

### (Item 14)

An operator-side apparatus comprising:
a surgical instrument operation unit to receive an operation amount for a surgical instrument attached to a distal end of a robot arm; and
a controller; wherein
the surgical instrument operation unit includes a board to which a signal received by the surgical instrument operation unit is input; and
the controller is connected to the board by serial communication via a wire line.

## Claims

1. A robotic surgical system comprising:
a surgical instrument operation unit to receive an operation amount for a surgical instrument attached to a distal end of a robot arm; and
a controller; wherein
the surgical instrument operation unit includes a first board to which a signal received by the surgical instrument operation unit is input; and
the controller is connected to the first board by serial communication via a first wire line.

2. The robotic surgical system according to claim 1, wherein
the surgical instrument operation unit includes a joint and a drive provided in the joint; and
the controller is connected to the first board by serial communication via the first wire line separately from a signal communication path between a detector operable to detect a movement amount of the drive and the controller.

3. The robotic surgical system according to claim 2, wherein the detector is connected to the controller by serial communication via a second wire line.

4. The robotic surgical system according to claim 1, wherein the controller is connected to the first board by serial communication through an inside of the surgical instrument operation unit.

5. The robotic surgical system according to claim 1, wherein the controller is connected to the first board by serial communication through a relay.

6. The robotic surgical system according to claim 5, wherein the relay is serially connected to at least one of the controller or the first board via the first wire line including flexible printed wiring.

7. The robotic surgical system according to claim 1, wherein
the surgical instrument operation unit includes a pair of grip members that are opened and closed by an operator, and an angle detection sensor to detect an opening angle between the pair of grip members; and
the first board is operable to receive an analog signal from the angle detection sensor.

8. The robotic surgical system according to claim 7, wherein the angle detection sensor is serially connected to the first board via the first wire line including flexible printed wiring.

9. The robotic surgical system according to claim 7, wherein
the surgical instrument operation unit further includes:
a first link on which the pair of grip members are placed; and
a second link connected to the first link; and
the first board is placed inside the first link or the second link.

10. The robotic surgical system according to claim 9, wherein
the surgical instrument operation unit further includes:
a third link connected to the second link;
a fourth link connected to the third link; and
an arm to support the fourth link; and
the controller is connected to the first board by serial communication via the first wire line through insides of the first link or the second link in which the first board is placed, the third link, the fourth link, and the arm.

11. The robotic surgical system according to claim 10, further comprising:
a first relay board in the third link;
a second relay board in the fourth link; and
a relay placed in the arm; wherein
the first board is connected to the first relay board via the first wire line including flexible printed wiring;
the first relay board is connected to the second relay board via the first wire line including the flexible printed wiring; and
the second relay board is connected to the relay via the first wire line including the flexible printed wiring.

12. The robotic surgical system according to claim 1, further comprising:
a main body to support the surgical instrument operation unit; wherein
the controller is placed inside the main body; and
the controller is connected to the first board by serial communication via the first wire line through insides of the surgical instrument operation unit and the main body.

13. The robotic surgical system according to claim 1, further comprising:
a foot pedal operated by a foot of an operator;
a foot detector to detect the foot of the operator that operates the foot pedal; and
a second board to which a signal from the foot detector is input; wherein
the controller is connected to the second board by serial communication via the first wire line.

14. An operator-side apparatus comprising:
a surgical instrument operation unit to receive an operation amount for a surgical instrument attached to a distal end of a robot arm; and
a controller; wherein
the surgical instrument operation unit includes a board to which a signal received by the surgical instrument operation unit is input; and
the controller is connected to the board by serial communication via a wire line.
